# EUROPEAN PATENT SPECIFICATION

(11) **EP 3 282 939 B1**
(45) Date of publication and mention of the grant of the patent: **03.03.2021**
(21) Application number: 16722948.3
(22) Date of filing: 13.04.2016
(51) Int. Cl.: A61B 5/0408, A61B 5/0428, A61B 5/0452, A61B 5/00

(54) **ALIGNMENT OF A DETACHABLE MONITORING DEVICE**
AUSRICHTUNG EINER ABNEHMBAREN ÜBERWACHUNGSVORRICHTUNG
ALIGNEMENT D'UN DISPOSITIF DE SURVEILLANCE AMOVIBLE

(30) Priority: 14.04.2015 US 201514685839; 14.04.2015 US 201514685866
(43) Date of publication of application: 21.02.2018
(73) Proprietor: LifeWatch Technologies, Ltd., 7670202 Rehovot (IL)
(72) Inventor: LEVANT, Anna, 7621017 Rehovot (IL); BEN-HAIM, Asher, Rehovot, 76100 Israel (IL); LOVTON, Yossi, 7405081 Nes Zeyona (IL)
(74) Representative: Graf von Stosch, Andreas
(86) International application number: PCT/IL2016/050391
(87) International publication number: WO 2016/166757

(56) References cited:
- WO-A1-91/00053
- WO-A1-2009/112977
- WO-A1-2009/147279
- WO-A1-2014/125159
- MERJA PUURTINEN ET AL: "Best Electrode Locations for a Small Bipolar ECG Device: Signal Strength Analysis of Clinical Data", ANNALS OF BIOMEDICAL ENGINEERING, KLUWER ACADEMIC PUBLISHERS-PLENUM PUBLISHERS, NE, vol. 37, no. 2, 20 November 2008 (2008-11-20), pages 331-336, XP019668304, ISSN: 1573-9686
- R Hoekema ET AL: "Interindividual variability of multilead electrocardiographic recordings: Influence of heart position", Journal of Electrocardiology, 1 January 1999 (1999-01-01), pages 137-148, XP055285592, UNITED STATES DOI: 10.1016/S0022-0736(99)90092-4 Retrieved from the Internet: URL:http://www.jecgonline.com [retrieved on 2016-07-06]
- SOTIRIOS NEDIOS ET AL: "Precordial electrode placement for optimal ECG monitoring: Implications for ambulatory monitor devices and event recorders", JOURNAL OF ELECTROCARDIOLOGY., vol. 47, no. 5, 1 September 2014 (2014-09-01), pages 669-676, XP055285892, XX ISSN: 0022-0736, DOI: 10.1016/j.jelectrocard.2014.04.003

## Description

### BACKGROUND OF THE INVENTION

During the last couple of years the importance of health monitoring has increased. Remote health monitoring allows a person being monitored to continue his/her daily routine without being restricted to hospitals.

When a monitor is used on a patient and intended to be worn during daily activities, it can be advantageous to have a smaller monitor - such that less adhesive is used, less of the body is contacted, and, generally, the monitor is less burdensome. There is a growing need to provide efficient health monitors that are compact and are accurate.

There is also a growing need to provide a robust and noise reducing electrode housing for electrodes of the monitoring device. WO 2014/125159 A1 provides means and methods for monitoring heart activity during physical exercise, whereby measurement configurations are chosen based on QRS complex waveforms. However, there is still a need for optimized monitoring devices and methods for aligning them.

### SUMMARY OF THE INVENTION

According to various embodiments of the invention there is provided a method for aligning a monitoring device and a computerized control unit.

According to various embodiments of the invention there is provided a method according to claim 1 for monitoring electrocardiographic activity of a person, the method includes performing a first set of alignment iterations for aligning a monitoring device and an electrical heart axis of the person; wherein each alignment iteration of the first set comprises: receiving, by a computerized control unit, electrocardiographic signals sensed by the monitoring device while the monitoring device is positioned at certain orientation while being detachably coupled to a chest of the person; determining, by the computerized control unit and based upon the electrocardiographic signals, whether the monitoring device is aligned with the electrical heart axis of the person; wherein the determining is responsive to amplitudes of a P wave sensed by the monitoring device and a ratio between amplitudes of a T wave sensed by the monitoring device and an R wave sensed by the monitoring device; wherein when determining that the monitoring device is aligned with the electrical heart axis then assisting, by the computerized control unit, in a provision of an alignment indicator to the person; and wherein when determining that the monitoring device is misaligned with the electrical heart axis then assisting, by the computerized control unit, in a provision of a request to change, by the person, an alignment of the monitoring device.

The method may include stopping of the performing of the first set of alignment iterations when determining that the monitoring device is aligned with the electrical heart axis.

The request may include an indication about a desired change of orientation of the monitoring device.

The method may include determining that the monitoring device is aligned with the electrical heart axis when a peak of a P wave sensed by the monitoring device exceeds an amplitude threshold and a ratio between a peak of a T wave sensed by the monitoring device and a peak of an R wave sensed by the monitoring device exceeds a ratio threshold.

The method includes selecting a selected orientation out of all orientations evaluated during the first set of alignment iterations in response to values of peaks of P waves, R waves and T waves sensed by the monitoring device during the alignment iterations of the first set of iterations, as described above.

The method may include assisting, by the computerized control unit, in a provision, to the person, of a request to (a) attach to the person an additional set of electrodes and (b) position the monitoring device below the chest of the person after each one of the alignment iterations of the first set of alignment iterations failed to align the monitoring device with the electrical heart axis.

Further described herein is a non- transitory computer readable medium that stores instructions that once executed by a computerized control unit causes the computerized control unit to perform a first set of alignment iterations for aligning a monitoring device and an electrical heart axis of a person; wherein each alignment iteration of the first set may include: receiving, by the computerized control unit, electrocardiographic signals sensed by the monitoring device while the monitoring device is positioned at certain orientation while being detachably coupled to a chest of the person; determining, by the computerized control unit and based upon the electrocardiographic signals, whether the monitoring device is aligned with the electrical heart axis of the person; wherein when determining that the monitoring device is aligned with the electrical heart then assisting, by the computerized control unit, in a provision of an alignment indicator to the person; and wherein when determining that the monitoring device is misaligned with the electrical heart then assisting, by the computerized control unit, in a provision of an request to change, by the person, an alignment of the monitoring device.

The non-transitory computer readable medium may store instructions for may include stopping the preforming of the first set of alignment iterations when determining that the monitoring device is aligned with the electrical heart.

The request may include an indication about a desired change of orientation of the monitoring device.

The determining may be responsive to amplitudes of a P wave sensed by the monitoring device and a ratio between amplitudes of a T wave sensed by the monitoring device and a R wave sensed by the monitoring device.

The non-transitory computer readable medium may store instructions for determining that the monitoring device is aligned with the electrical heart axis when a peak of a P wave sensed by the monitoring device exceeds an amplitude threshold and a ratio between a peak of a T wave sensed by the monitoring device and a peak of a R wave sensed by the monitoring device exceeds a ratio threshold.

The non-transitory computer readable medium may store instructions for selecting a selected orientation out of all orientations evaluated during the first set of alignment iterations in response to values of peaks of P waves, R waves and T waves sensed by the monitoring device during the alignment iterations of the first set of iterations.

The non-transitory computer readable medium may store instructions for assisting, by the computerized control unit, in a provision, to the person, of a request to (a) attach to the person an additional set of electrodes and (b) position the monitoring device below the chest of the person after each one of the alignment iterations of the first set of alignment iterations failed to align the monitoring device with the electrical heart axis.

According to various embodiments of the invention there is provided a computerized control unit according to claim 7 that includes a transceiver, a man machine interface and an electrocardiographic processor, wherein the computerized control unit is configured to perform a first set of alignment iterations for aligning a monitoring device and an electrical heart axis of a person; wherein each alignment iteration of the first set includes: receiving, by the transceiver, electrocardiographic signals sensed by the monitoring device while the monitoring device is positioned at certain orientation while being detachably coupled to a chest of the person; determining, by the electrocardiographic processor and based upon the electrocardiographic signals, whether the monitoring device is aligned with the electrical heart axis of the person; wherein the determining, by the electrocardiographic processor, is responsive to amplitudes of a P wave sensed by the monitoring device and a ratio between amplitudes of a T wave sensed by the monitoring device and an R wave sensed by the monitoring device; wherein when determining that the monitoring device is aligned with the electrical heart then assisting, by at least one of the man machine interface or the transceiver, in a provision of an alignment indicator to the person; and wherein when determining that the monitoring device is misaligned with the electrical heart axis then assisting, by at least one of the man machine interface or the transceiver, in a provision of an request to change, by the person, an alignment of the monitoring device. The computerized control unit may be a computer, a mobile phone, a desktop computer, a laptop computer, a personal data assistant, and the like.

The man machine interface may be configured to provide the alignment indicator to the person.

The computerized control unit wherein the transceiver may be configured to transmit the alignment indicator to the monitoring device.

The electrocardiographic processor may be configured to stop the preforming of the first set of alignment iterations when determining that the monitoring device is aligned with the electrical heart.

The request may include an indication about a desired change of orientation of the monitoring device.

The electrocardiographic processor may be configured to determine that the monitoring device is aligned with the electrical heart axis when a peak of a P wave sensed by the monitoring device exceeds an amplitude threshold and a ratio between a peak of a T wave sensed by the monitoring device and a peak of an R wave sensed by the monitoring device exceeds a ratio threshold.

The electrocardiographic processor is configured to select a selected orientation out of all orientations evaluated during the first set of alignment iterations in response to values of peaks of P waves, R waves and T waves sensed by the monitoring device during the alignment iterations of the first set of iterations, as described above.

The man machine interface may be configured to provide, to the person, of a request to (a) attach to the person an additional set of electrodes and (b) position the monitoring device below the chest of the person after each one of the alignment iterations of the first set of alignment iterations failed to align the monitoring device with the electrical heart axis.

The transceiver may be configured to transmit, to the monitoring device, a request to (a) attach to the person an additional set of electrodes and (b) position the monitoring device below the chest of the person after each one of the alignment iterations of the first set of alignment iterations failed to align the monitoring device with the electrical heart axis.

Further described herein is a monitoring device that may include a detachable processing and transmitting module, multiple electrodes for sensing electrocardiogram signals; an elastic layer that comprises multiple electrode housings for supporting the multiple electrodes; wherein each electrode housing that is made of an elastic material and comprises a trench that includes an exterior sidewall, a bottom and an internal sidewall; a group of structural elements; wherein the internal sidewall is coupled to the group of structural elements; wherein the group of structural elements is surrounded by the trench and defines a cavity that matches a shape and size of an electrode.

Further described herein is an electrode housing that is made of an elastic material and may include a trench that includes an exterior sidewall, a bottom and an internal sidewall; a group of structural elements; wherein the internal sidewall is coupled to the group of structural elements; wherein the group of structural elements is surrounded by the trench and defines a cavity that matches a shape and size of an electrode. The trench and the group of structural elements may be integrated.

The depth of the trench may be few millimeters.

The trench and the group of structural elements may be radially symmetric.

The group of structural elements may include an annular segmented base and an inner annular cover that define the cavity.

The group of structural elements may include an external ring and an inner trench that is delimited between the inner annular cover and the external ring.

The annular segmented base may define an opening that is shaped and sized to match an upper part of an electrode.

The elastic material can be thermoplastic polyurethane.

### BRIEF DESCRIPTION OF THE DRAWINGS

The foregoing and other objects, features, and advantages of the present invention will become more apparent from the following detailed description when taken in conjunction with the accompanying drawings. In the drawings, similar reference characters denote similar elements throughout the different views, in which:
FIG. 1 illustrates a person wearing a monitoring device positioned at a first orientation on the chest of the person according to an embodiment of the invention;
FIG. 2 illustrates a person wearing a monitoring device positioned at a second orientation on the chest of the person according to an embodiment of the invention;
FIG. 3 illustrates a person wearing a monitoring device positioned at a third orientation on the chest of the person according to an embodiment of the invention;
FIG. 4 illustrates a person wearing a monitoring device positioned at a fourth orientation on the chest of the person according to an embodiment of the invention;
FIG. 5 illustrates a person wearing a monitoring device positioned at a fifth orientation on the chest of the person according to an embodiment of the invention;
FIG. 6 illustrates a person wearing a monitoring device positioned at an orientation below the mammary tissue of the right breast chest with at least one additional electrode that are coupled to the monitoring device as described herein;
FIG. 7 is a flow chart of a method according to an embodiment of the invention;
FIG. 8 illustrates a person wearing a monitoring device, a computerized control unit, a cellular network base station, and a remote monitoring system as described herein;
FIG. 9 is a screenshot of a display of the computerized control unit of FIG. 8 according to an embodiment of the invention;
FIG. 10 is a flow chart of a method according to an embodiment of the invention;
FIG. 11 1s a flow chart of a method according to an embodiment of the invention;
FIG. 12 illustrates a top part of the housing of the monitoring device as described herein;
FIG. 13 is an exploded view of the monitoring device as described herein;
FIG. 14 is a block diagram of the monitoring device as described herein;
FIG. 15 illustrates the monitoring device with additional electrodes as described herein;
FIG. 16 illustrates an elastic layer of the monitoring device as described herein;
FIG. 17 illustrates a portion of an elastic layer of the monitoring device as described herein;
FIG. 18 is a cross sectional view of a portion of an elastic layer of the monitoring device as described herein; and
FIG. 19 is a cross sectional view of a portion of an elastic layer of the monitoring device, of an electrode and other components of the monitoring device as described herein.

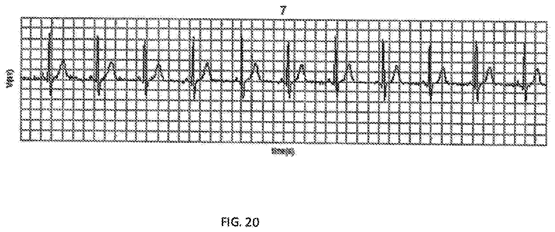

FIG. 20 illustrates a non-limiting example of the monitoring device as described herein.

### DETAILED DESCRIPTION OF THE DRAWINGS

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

The subject matter regarded as the invention is particularly pointed out and distinctly claimed in the concluding portion of the specification. The invention, however, both as to organization and method of operation, together with objects, features, and advantages thereof, may best be understood by reference to the following detailed description when read with the accompanying drawings.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

Because the illustrated embodiments of the present invention may for the most part, be implemented using electronic components and circuits known to those skilled in the art, details will not be explained in any greater extent than that considered necessary for the understanding and appreciation of the underlying concepts of the present invention and in order not to obfuscate or distract from the teachings of the present invention.

Any reference in the specification to a method should be applied mutatis mutandis to a system capable of executing the method and should be applied mutatis mutandis to a non-transitory computer readable medium that stores instructions that once executed by a computer result in the execution of the method.

Any reference in the specification to a system should be applied mutatis mutandis to a method that may be executed by the system and should be applied mutatis mutandis to a non-transitory computer readable medium that stores instructions that may be executed by the system.

Any reference in the specification to a non-transitory computer readable medium should be applied mutatis mutandis to a system capable of executing the instructions stored in the non-transitory computer readable medium and should be applied mutatis mutandis to method that may be executed by a computer that reads the instructions stored in the non-transitory computer readable medium.

### Alignment of monitoring device to electrical heart axis.

The present invention relates to placement and orientation of a monitoring device wherein the placement aligns the monitoring device with the electrical heart axis. Such alignment may be particularly useful when the monitoring device is compact. When placing a monitor, it is useful to align the monitoring device with the electrical heart axis of that person. This can be more challenging with more compact devices because there typically is less distance between electrodes on a compact device. When the electrodes are more proximate, the amplitude is typically about ¼ less than the measurement when the electrodes are more distal from one another. There is also a growing need to provide a monitoring device installation method that can be executed by a person and allow the person to position the monitoring device at a desired orientation.

A compact device may have a relatively small surface area such that electrodes placed thereon are proximate one another. For example, the monitoring device 100 may have a length L of 5-15 centimeters, a width W of 4-9 centimeters, and a height H of 1.4-1.7 centimeters. In a specific embodiment, the monitoring device 100 may have a length L of 15 centimeters, a width W of 8 centimeters, and a height H of 1.65 centimeters. It is to be appreciated, however, that the methods taught herein may be useful for placement of a monitoring device of sizes widely variant from the specific ranges discussed above. Further, while the methods taught herein may be especially useful for compact monitoring devices because of the of the low amplitudes resulting from such devices, they work equally well for larger monitoring devices.

The monitoring device may have three electrodes, referred to as first, second, and third electrodes. In some embodiments, the distance between these electrodes may be few centimeters.

According to an embodiment of the invention there is provided a method and a computerized monitoring unit that assist in placement of a monitoring devices by aligning the monitoring device with the electrical heart axis.

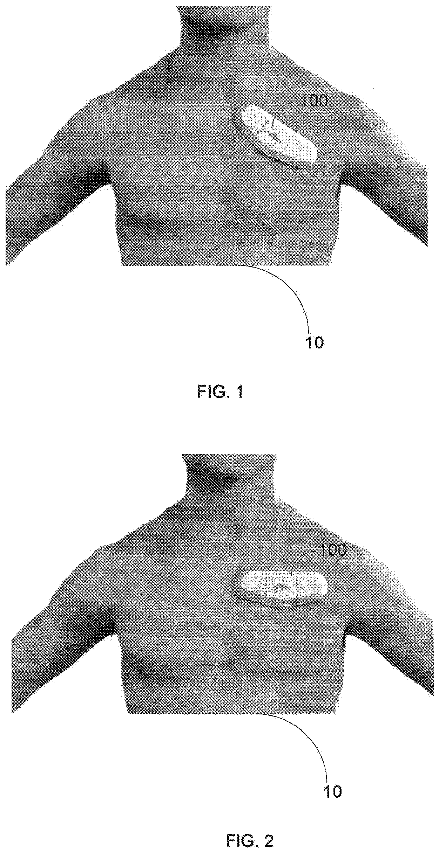

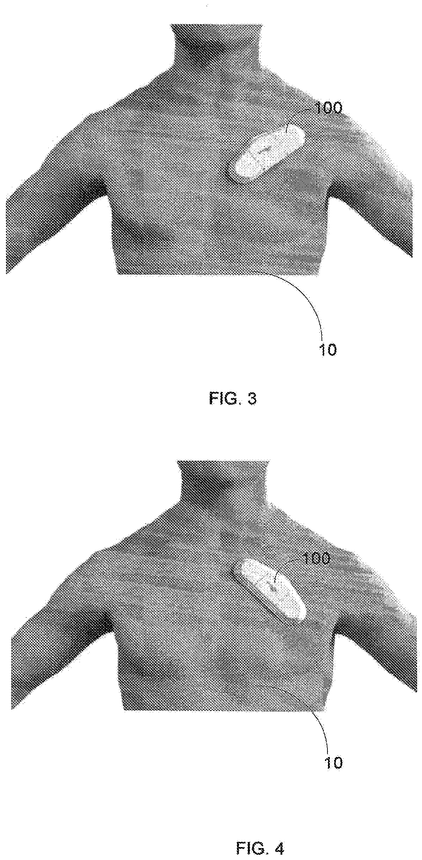

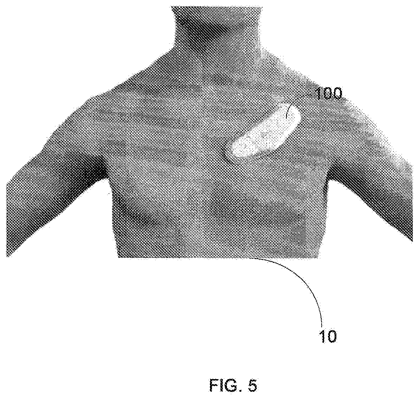

The alignment process includes multiple alignment iterations. Figures 1-5 illustrate an alignment process that includes five alignment iterations, wherein the monitoring device is placed on the chest over the left breast.

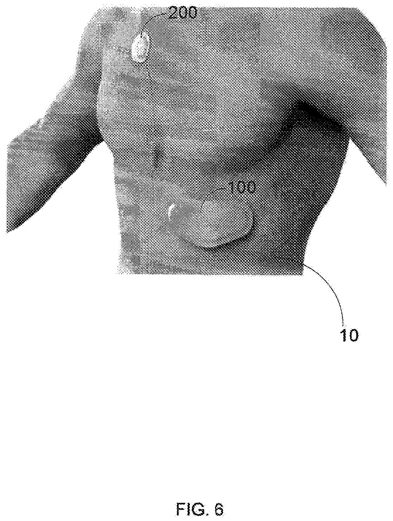

The five alignment iterations form a first set of alignment iterations. If all the alignment iterations of the first alignment iterations fail (an adequate alignment was not obtained) then the first set of alignment iterations is followed by placement in the orientation shown in Figure 6. In this orientation, the monitoring device is placed on the left of the person towards the abdomen, below mammary tissue, and additional electrodes are attached in a position on the chest over the right breast.

The specifics of the methods disclosed are merely illustrative. The number of alignment iterations may differ from five - may exceed five or be less than five - and that five alignment iterations is merely one example. The alignment process may stop the execution of the alignment iterations when finding an alignment. For example - if in the third alignment iteration of the five alignment iterations of Figure 1-5 an alignment is found that achieves suitable results, the alignment process may stop and the fourth and fifth alignment orientations may not be performed.

Similarly, the rotations of the monitoring device between adjacent alignment iterations may be substantially even or may differ from each other. The alignment process may execute a certain amount of alignment iterations regardless of the results of the alignment iterations and then determine a selected position of the monitoring device.

The iteration process may include a conversion phase during which one or more orientations are evaluated, wherein the angular differences between evaluated orientations of the convergence phase are smaller than the angular differences between orientations of alignment iterations that preceded the convergence phase.

Any change of the orientation of the monitoring device may also include a non-rotational change in the position of the monitoring device.

Figure 1 illustrates a person 10 wearing a monitoring device 100 positioned on the left chest over the mammary tissue. The monitoring device 100 is a compact mobile electrocardiographic system, which is portable by a medical practitioner or a first aid provider, in order to quickly diagnose a person, or may be used for extended wearing of a person either for analysis of or for monitoring his condition. The monitoring device 100 may have the ability to transport gathered data wirelessly to facilitate monitoring by a medical center.

The method for aligning the monitoring device with the electrical heart axis of the person may be beneficial to compact monitoring devices having relatively small distances between electrodes but may also be used for larger monitoring devices. In some embodiments, the monitoring device 100 may be equipped with sensors that differ from electrode based sensors (for example light and/or infrared based sensors - such as oximeters) and the monitoring device 100 may be arranged to monitor each physiological signal of the person. Other sensors may be added. Adding more sensors may require, for example, adding appropriate apertures in the bottom layer of the monitoring device 100 as may be appreciated by one skilled in the art.

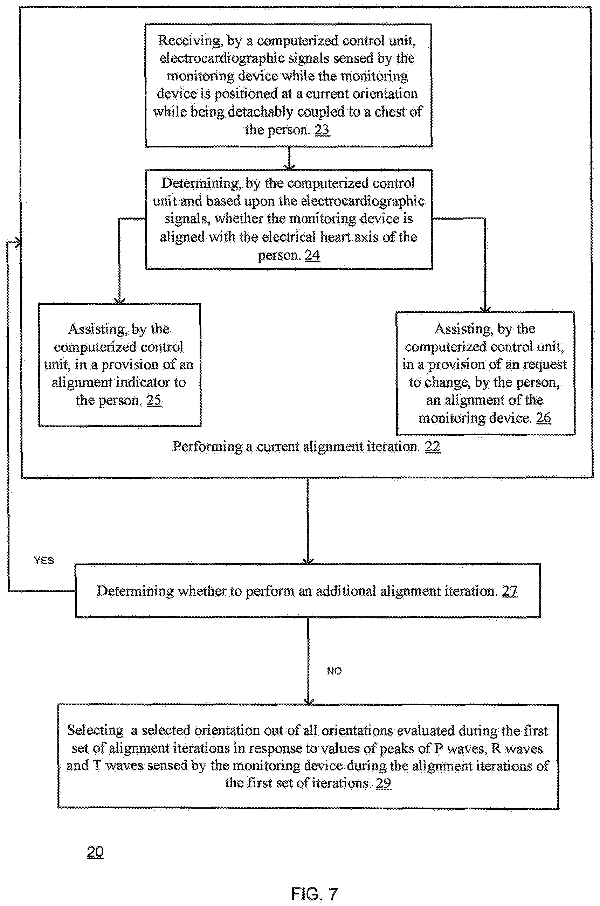

Figure 7 illustrates method 20 according to an embodiment of the invention. The steps shown in method 20 reflect the iterative alignment shown in Figures 1-5.

Method 20 begins with performing a current aligni-nent iteration [step 22] and may include one or more of steps 23, 24, 25, and 26.

Step 23 may include receiving, by a computerized control unit, electrocardiographic signals sensed by the monitoring device while the monitoring device is positioned at a current orientation while being detachably coupled to a chest of the person.

Step 23 may be followed by determining, by the computerized control unit and based upon the electrocardiographic signals, whether the monitoring device is aligned with the electrical heart axis of the person [step 24].

The invention utilizes the methodology described herein for determining whether the monitoring device is aligned with the electrical heart axis [step 24], and such methodology generally including detecting P waves, R waves and T waves. Determining whether the monitoring device is aligned with the electrical heart axis is responsive to amplitudes of a P wave sensed by the monitoring device and a ratio between amplitudes of a T wave sensed by the monitoring device and an R wave sensed by the monitoring device. An amplitude of a wave (R wave, T wave P wave) may include a peak of the wave, an average of the wave or an outcome of any other mathematical function applied on amplitudes of the wave during one or more points in time.

Step 24 includes determining that the monitoring device is aligned with the electrical heart axis when a peak of a P wave sensed by the monitoring device exceeds an amplitude threshold and a ratio between a peak of a T wave sensed by the monitoring device and a peak of a R wave sensed by the monitoring device exceeds a ratio threshold.

If it is determined in step 24 that the monitoring device is aligned with the electrical heart, step 24 is followed by assisting, by the computerized control unit, in a provision of an alignment indicator to the person [step 25]. Step 25 may alternatively be referred to as providing an alignment indicator.

Providing an alignment indicator generally means giving some indication to the person doing the alignment that alignment has been achieved. For example, providing an alignment indicator may include providing an alignment indicator such as generating an audio indicator, generating a visual indicator, triggering the monitoring device to generate the alignment indicator, and the like.

In contrast, if the result of step 24 is determining that the monitoring device is misaligned with the electrical heart axis then step 24 is followed by assisting, by the computerized control unit, in a provision of a request to change, by the person, an alignment of the monitoring device [step 26]. Step 26 may alternatively be referred to as providing a request to change an alignment of the monitoring device.

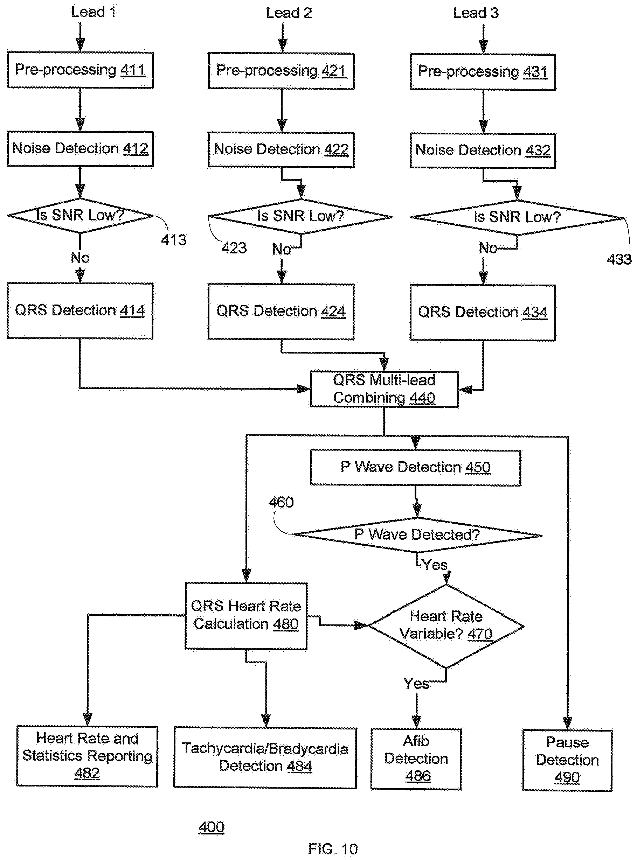

The request to change an alignment of the monitoring device may include an indication about a desired change of orientation of the monitoring device. Figure 10 illustrates a screenshot of a display of a computerized control unit displaying either the current position of the monitoring device or a requested new position of the monitoring device.

Providing a request to change the alignment of the device may include generating an audio indicator, generating a visual indicator, triggering the monitoring device to generate the alignment indicator, and the like.

Returning to Figure 7, performing a current alignment iteration [step 22] includes one or more of steps 23, 24, 25, and 26. Performing a current alignment iteration [step 22] may be followed by determining whether to perform an additional alignment iteration [step 27].

In some embodiments, a predefined number of alignment iterations may be performed. For example, in some embodiments, it may be recommended to perform five (5) alignment iterations. It is to be appreciated that using five as a number for predefined amount of alignment iterations is for illustrative purposes only and other numbers of alignment iterations may be performed. These alignment iterations may be referred to as a first set of alignment iterations.

Assuming that a predefined number of alignment iterations is recommended, determining whether to perform another alignment iteration [step 27] may comprise evaluating whether the predefined number of alignment iterations have been performed.

If the predefined number of alignment iterations have not been performed and an alignment between the monitoring device and the electrical heart axis was not achieved, then the result of step 27 may be to perform another alignment iteration.

If the predefined number of alignment iterations was performed and an alignment between the monitoring device and the electrical heart axis was not achieved, the result of step 27 may be to proceed to assisting in a provision of a request to position the monitoring device at a certain position, attach additional electrodes to the person and couple the additional electrodes to the monitoring device [step 30]. Step 30 may also be referred to as providing a request to (a) position the monitoring device at an alternate location (for example, see Figure 6), (b) position additional electrodes on the person, and (c) couple the additional electrodes to the monitoring device.

Regardless of whether the predefined number of alignment of iterations have been performed, if one of the alignment iterations resulted in an alignment between the monitoring device and the electrical heart axis, step 27 may result in a determination not to perform another alignment iteration. This can be the last alignment iteration that was executed or one of the previously execute alignment iterations.

Upon a determination not to perform another alignment iteration in step 27, one may proceed with selecting a selected orientation out of all orientations evaluated during the first set of alignment iterations [step 29]. Such selection may be in response to values of peaks of P waves, R waves and T waves sensed by the monitoring device during the alignment iterations of the first set of iterations. Step 29 may include assisting, by the computerized control unit, in a provision of an request to change, by the person, an alignment of the monitoring device to the selected orientation - if the selected alignment differs from the current alignment of the monitoring device.

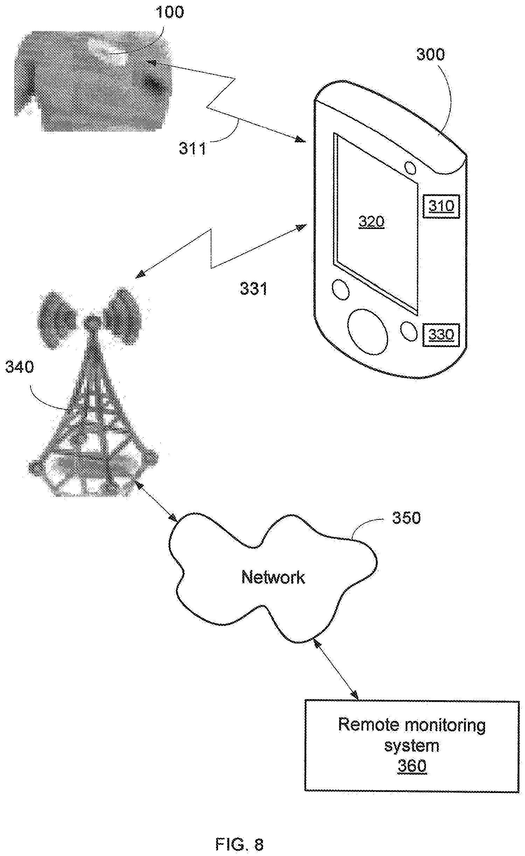

Figure 8 illustrates a monitoring device 100 that is detachably attached to a person 10, a computerized control unit 300, a cellular base station 340, a network 350 and a remote monitoring system 360 . The computerized control unit 300 wirelessly communicates via a wireless link 311 with monitoring device 100. The computerized control unit 300 wirelessly communicates with a remote monitoring system 360 via wireless link 331, cellular base station 340 and network 350. Network 350 may be the Internet or any other wired and/or wireless network.

In some embodiments, the wireless link 311 may be a short range communication link and the wireless link 331 may be a long range communication link. However, any type of communication links and/or communication protocols may be used to convey information between monitoring device 100, computerized control unit 300 and remote monitoring system 360.

The computerized control unit 300 may be a personal data accessory, a mobile phone, a laptop computer, a desktop computer, a tablet, a media player, and the like. The exact configuration of the computerized control unit 300 may vary so long as it is capable of executing method 20.

According to one embodiment, the computerized control unit 300 includes a transceiver 310, a man machine interface 320, and an electrocardiographic processor 330. The electrocardiographic processor 330 may be any type of hardware processor that is configured to execute instructions for processing electrocardiographic signals sensed by monitoring device 100.

The man machine interface may be a display, a touch screen, an audio generator, one or more control buttons, or any other man machine interface known in the art.

The computerized control unit 300 may be configured to perform a first set of alignment iterations for aligning a monitoring device and an electrical heart axis of the person. These alignment iterations may correspond to those described in reference to Figure 7.

Each alignment iteration of the first set may include: (I) receiving, by the transceiver 310, electrocardiographic signals sensed by the monitoring device while the monitoring device is positioned at a certain orientation on the person; (II) determining by the electrocardiographic processor 330 and based upon the electrocardiographic signals, whether the monitoring device is aligned with the electrical heart axis of the person; wherein the determining is responsive to amplitudes of a P wave sensed by the monitoring device and a ratio between amplitudes of a T wave sensed by the monitoring device and an R wave sensed by the monitoring device; (III) wherein when determining that the monitoring device is aligned with the electrical heart then assisting, by at least one of the man machine interface 320 or the transceiver 310, in a provision of an alignment indicator to the person; (IV) wherein when determining that the monitoring device is misaligned with the electrical heart axis then assisting, by at least one of the man machine interface 320 or the transceiver 310, in a provision of an request to change, by the person, an alignment of the monitoring device.

The electrocardiographic processor 330 may be configured to receive electrocardiogram (ECG) data from the monitoring device 100 and to detect events such as but not limited to heart pause, Arial Fibrillation, Tachycardia and Bradycardia. An occurrence of an event can be transmitted to the remote monitoring system 360. The remote monitoring system 360 may be staffed by medical personnel.

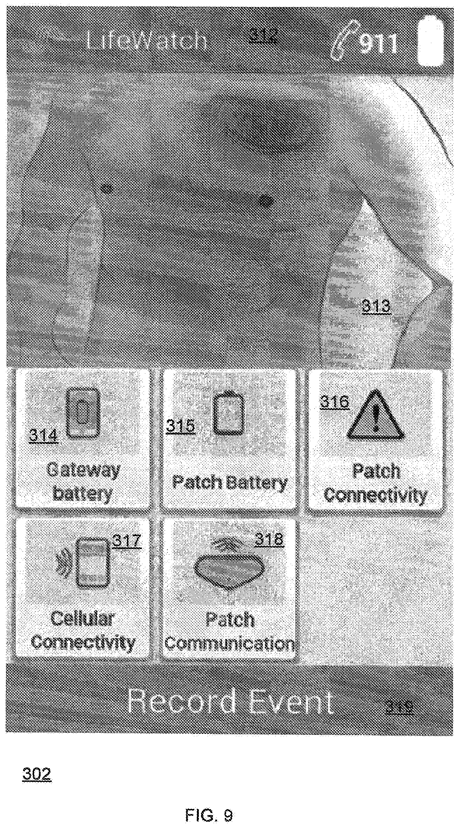

Figure 9 is a screenshot 302 of a display of the computerized control unit 300 according to an embodiment of the invention.

The screenshot 302 includes the following segments: (i) a top segment 312 that is illustrated as including a quick link to an emergency call "911", (ii) a main segment 313 that illustrates the torso of the person and a current (estimated) position of the monitoring device or a next (requested) position of the monitoring device, wherein an icon that represents the monitoring device may indicate whether the displayed location is the current location or the next location, (iii) status segments indicative of a status of a battery of the computerized control unit ("gateway battery" segment 314), of a status of a battery of the monitoring device ("patch battery" segment 315), of a status of connectivity (contact between person and electrodes) of the monitoring device ("patch connectivity" segment 316) that may indicate about the manner in which the monitoring device is coupled to the person, status of communication between the computerized control unit and the mobile base station ("cellular connectivity" segment 317), status of communication between the monitoring device and the computerized control unit ("patch communication" segment 319).

The arrangement of the screen shot as well as the specific events or statuses being reported on the display may differ from those illustrated in Figure 9.

Figure 10 illustrates a method 400 according to an embodiment of the invention. In general, the software application installed in the computerized control unit 300 analyzes the ECG data from the three electrodes 131 for cardiac arrhythmias. If an arrhythmia is detected, the computerized control unit 300 transmits a physiological alarm to the remote monitoring system 360. The transmitted information may include the cardiac arrhythmia type, event initiation timestamp, raw ECG data, and event termination timestamp.

In addition to analyzing the data and sending arrhythmia events, the computerized control unit 300 may have an option to continuously send the recorded ECG data files to the remote monitoring system 360. The computerized control unit 300 may analyze the ECG data for cardiac arrhythmias. If an arrhythmia is detected, the computerized control unit 300 transmits a physiological alarm to the remote monitoring system. The transmitted information may include includes the cardiac arrhythmia type, event initiation timestamp, raw ECG data, and event termination timestamp.

The computerized control unit 300 can detect cardiac arrhythmia events such as Pause, Atrial Fibrillation, Tachycardia, and Bradycardia (according to the device trigger settings). These cardiac arrhythmia events are intended to be illustrative only and other cardiac arrhythmia events that could be determined based on the collected data may alternatively be detected.

The computerized control unit 300 can perform the analysis using parameters that can be modified by a qualified technician, within an allowable range per request.

Returning now to Figure 10, the monitoring device may have first, second, and third electrodes. The first, second, and third electrodes measure ECG signals. The ECG signals from the first, second and third electrodes are processed by three corresponding sequences of steps.

The ECG signals from the first electrode are processed by pre-processing [step 411], noise detection [step 412], checking if the signal-to-noise ratio (SNR) is low [step 413], and step QRS detection [step 414].

The ECG signals from the second electrode are processed by pre- processing [step 421], noise detection [step 422], checking if the SNR is low [step 423], and QRS detection [step 424].

The ECG signals from the third electrode are processed by pre-processing [step 431], noise detection [step 432], checking if the SNR is low [step 433], and QRS detection [step 434].

QRS detection [steps 414, 424, and 434] is followed by QRS multi-lead combining [step 440] for merging the information obtained from the three electrodes.

QRS multi-lead combining [step 440] P wave detection [step 450, pause detection [step 490], and QRS heart rate calculation [step 480].

The steps after multi-lead combining [step 440] can generally be referred to as the analysis portion of the method 400. The analysis portion of method 400 of Figure 10 is geared to detecting specific characteristics of the heart.

The calculated heart rate resulting from the QRS heart rate calculation [step 480] may be used in one or more of heart rate and statistics reporting [step 482], Tachycardia and/or Bradycardia detection [step 484], and step 470 is checking if the heat rate is variable [step 470]. If the heart rate is determined as variable, Atrial Fibrillation (Afib) detection [step 486] may be done.

Accordingly, the method 400 shown in Figure 10 may detect Pause, Tachycardia and Bradycardia based on QRS analysis and detect Atrial Fibrillation based on P Wave detection.

More detail will now be given to each of the steps of Figure 10. It is to be appreciated that other manners of performing each step may be used so long as they are suitable for use with a monitoring device.

Pre-processing [steps 411, 421 and 431] may include drift and soft noise removal. The power supply of the computerized control unit may cause a ubiquitous electrical interference at the frequency of 50/60Hz. The pre-processing may eliminate that interference by using a notch filter. Human respiration also induces interference at very low frequency. This interference may be removed by the pro-processing by applying a high pass filter with a cut-off at approximately 0.05 Hz. The ECG signal can be corrupted by electromyography (EMG), or a muscle contraction wave, which is generated during various body movements. In the ECG Analysis Algorithm, this type of noise is reduced with a low pass filter that removes the EMG, but has minimal effect on the ECG signal.

Noise detection [steps 412, 422 and 432] may include detecting noise using the distribution of the first and second derivatives of the ECG signals. The patterns that are identified as strong noise are marked and ignored in the QRS detection phase.

QRS detection [steps 414, 424, and 434] may include extracting and marking QRS positions from the ECG signals. The detection may be based on the morphological characteristic of the QRS. QRS usually has a higher slope compared to other signals in the ECG.

QRS multi-lead combining [step 440] may include combining data from the three electrodes to arrive at a single R timing value for the QRS. The morphology of the QRS extracted from each electrode may be analyzed for signal quality. The signals from each electrode may be weighted based on the signal quality and combined to provide a single value for the R timing.

QRS heart rate calculation [step 480] may calculate the average heart rate in a sliding window of a pre-defined length to decrease the effect of noise on the QRS timing. The time between Rs in the QRS complexes, denoted as RR, is measured. The average value for the RRs in the sliding window is the calculated QRS heart rate. The standard deviation between RR lengths is also calculated.

P wave detection [steps 450 and 460] may include detecting the contraction of the atria, denoted as a P wave. P waves may be detected by locating local maxima and minima preceding QRS complexes. To ensure the detection of the P wave, this step may search for a series of similar waves located at the same distance from the QRS complex. Additionally, these P waves should have the same average interval as the corresponding RRs.

Pause detection [step 490] may be done by checking checks whether the heart ceases to beat for more than a pre-defined time. This may comprise measuring the time following a QRS in which no new QRS was detected. When a QRS is detected, this step may ensure that confidence in the QRS is high. If confidence is high, the step resets and restarts a timer. When the timer reaches a pre-defined threshold, a pause event is detected.

Tachycardia detection [step 484] may comprise detecting whether the heart rate exceeds a programmable value (Theart_rate_threshold) for a minimum threshold time.

Bradycardia detection [step 484] may comprise detecting whether the heart rate is below a programmable value (Bheart_rate_threshold) for a minimum threshold time.

Atrial fibrillation detection [step 486] may comprise detecting when RR intervals (duration between successive beats) are of variable length and an absence of P-waves.

Step 486 may include detecting atrial fibrillation by the presence of an irregular heart rate and the absence of P waves. Weighted values are assigned to irregular heart rate and absence of P waves based on the confidence level in the results from the QRS heart rate calculation and P Wave detection modules. The weighted values are then combined to determine the atrial fibrillation grade. If the grade exceeds a pre-defined threshold, atrial fibrillation is detected.

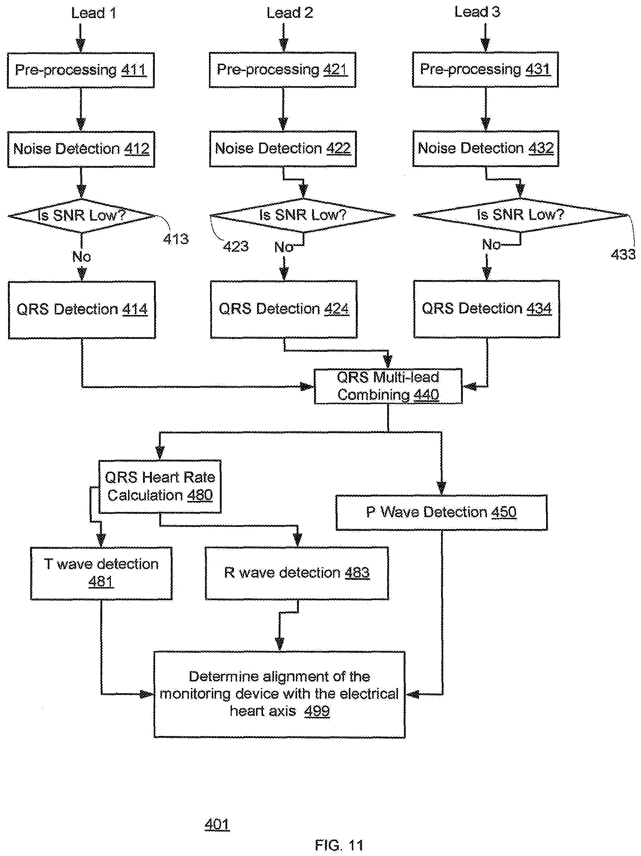

Figure 11 illustrates a method 401 according to an embodiment of the invention. Method 401 of Figure 11 differs from method 400 of Figure 10 in the analysis portion of the method (the steps after multi-lead combining [step 440]). The analysis portion of method 401 of Figure 10 is geared to placement of the monitoring device by determining alignment of the monitoring device with the electrical axis of the heart.

Like method 400 of Figure 10, method 401 includes pre-processing [steps 411, 421 and 431], noise detection [steps 412, 422 and 432], determining if the SNR is low [steps 413, 423 and 433], QRS detection [steps 414, 424 and 434], multi-lead combining 440], and QRS heart rate detection [step 480]. The analysis portion of method 401 then includes T wave detection [step 481], R wave detection [step 483], and determining alignment of the monitoring device with the electrical axis of the heart [step 499].

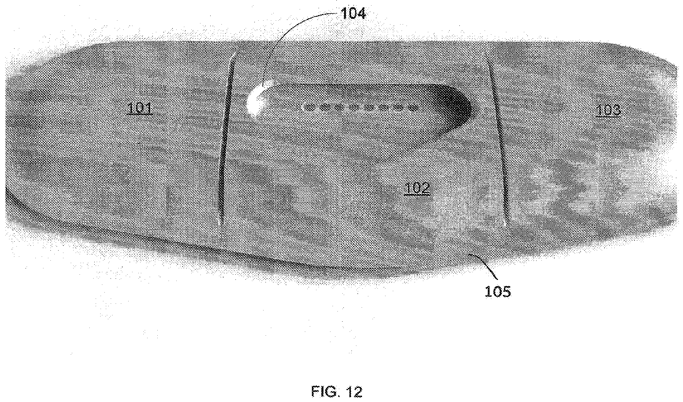

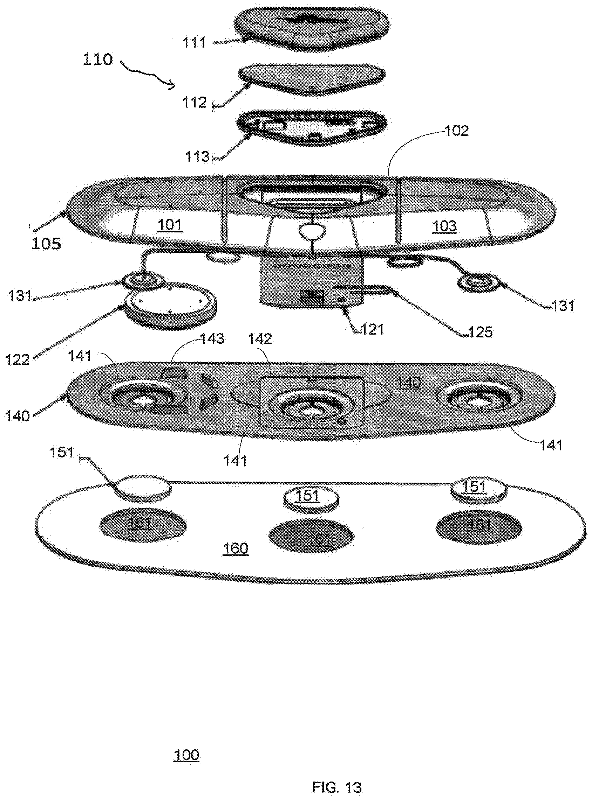

Figures 12 and 13 illustrate a monitoring device 100. The device includes a disposable housing 105 and a processing and transmitting module 110. The processing and transmitting module 110 is removable and can be re-used multiple times while other parts of the monitoring device 100 are disposable.

Figure 13 is an exploded view of the monitoring device 100. The processing and transmitting module 110 includes a top cover 111, a bottom cover 113 and electronics (including a transceiver and a processor) 112 that are positioned between the top cover 111 and bottom cover 113.

In the embodiment shown, monitoring device 100 includes a top cover that comprises a left top cover portion 101, a middle top cover portion 102, and a right top cover portion 103. The middle top cover portion 102 is positioned between the left top cover portion 10 and the right top cover parts 103. The middle top cover portion includes an opening 104. The processing and transmitting module 110 may be positioned in the opening 104.

The monitoring device 100 further includes a base layer 140 that is positioned above a bottom layer 160. The bottom layer 160 may be flexible but may alternatively be rigid. The bottom layer 160 includes three recesses 161.

Hydrogel elements 151 are positioned within the three recesses so that the bottom of the hydrogel elements 151 contact the person when the bottom layer 160 is coupled to the person. It is noted that the bottom layer 160 may include adhesive elements for attaching the monitoring device 100 to the person.

The base layer 140 has three electrode housing 141, a battery interface 143 (for supporting and holding battery 122) and a processing and transmitting module interface 142 that contacts and supports the processing and transmitting module 110.

Base layer 140 is also connected to a printed circuit board 121 that may be connected to sockets (not shown).

The base layer 140 and especially the electrode housings 141 of the base layer 140 can be made of an elastic material such as but not limited to thermoplastic polyurethane (TPU). The TPU may be any polyurethane plastic.

Three electrodes 131 (out of which two electrodes are shown) are positioned within the three electrode housing 141 and their bottom is contacted by hydrogel elements 151. Figure 13 illustrates three cables (including cable 125) that couples the printed circuit board 121 to the three electrodes.

The three electrodes 131 are used to provide for 1 lead arrhythmia detection.

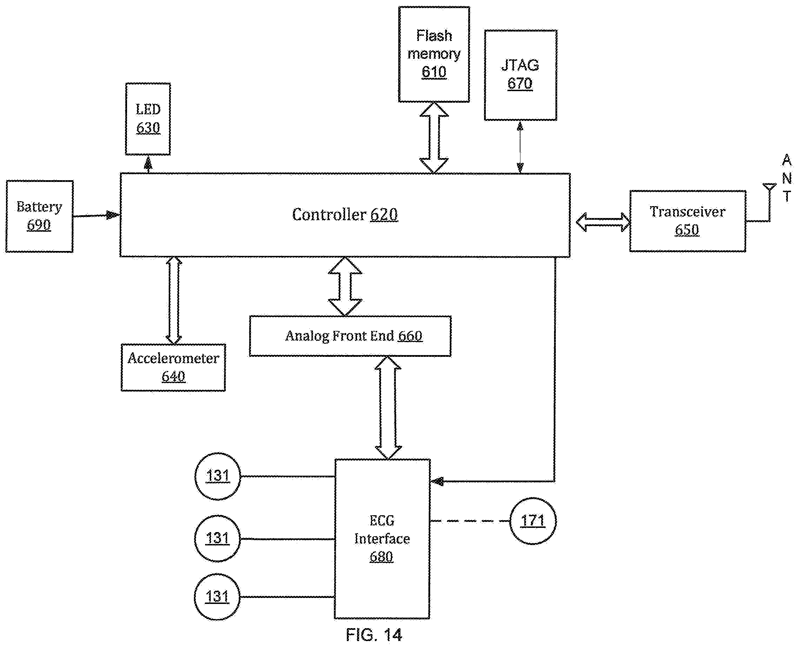

Figure 14 is a block diagram of the monitoring device 100 .

The processing and transmitting module 110 is illustrated as including a controller 620 and a transceiver 650 that is coupled between the controller 620 and an antenna. The processing and transmitting module 110 is detachable.

The controller 620 is configured to control the monitoring device 100 and may be configured to perform pre-processing (such as filtering) of the physiological signals sensed by the monitoring device 100. The preprocessing may include digital and/or filtering. The detection signals from the electrodes (either pro-processed or not) are stored in flash memory 610 and transmitted by transceiver 650 to the computerized control unit. The flash memory 610 can store several hours of detection signals.

The controller 620 is coupled to a light emitting diode (LED) 630, to battery 690, to a memory unit such as flash memory 610, to a JTAG interface 670, to an accelerometer 640, to an analog front end 660 and to an ECG interface 680.

The controller 620 may monitor the status of the battery 690 and send status indications (including a battery empty status alert) to the computerized control unit 300.

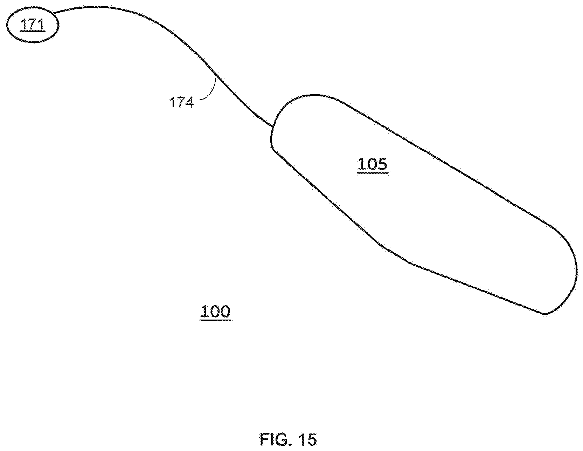

The ECG interface 680 is further coupled to electrodes 131 and to the analog front end 660. The analog front end 660 may also be coupled to an additional electrode 171 (also illustrated in figure 15).

The ECG interface 680 may provide the ECG signals from the electrodes 131 (and/or 171) and provide them to analog front end 660.

The analog front end 660 may process the ECG signals in the analog domain (for example - perform filtering operations) and perform an analog to digital conversion of the ECG signals to provide digital ECG signals. The analog front end 660 and/or the controller 620 may digitally processes digital ECG signals.

The analog front end 660 may be configured to test whether there is a proper connection with the electrodes 131 (for example by monitoring signals provided from the electrodes or lack of such signals or by sending a low current and testing the impedance) and to trigger the controller 620 to send an alert (to the computerized control unit 300) in case that one or more electrodes are not properly connected.

The JTAG interface 670 may be used for testing the controller 620 and/or for downloading firmware or software to the controller 620.

In case of communication failures error the monitoring device 100 may wait till the recovery of the communication and then transmit the ECG data that was gained during the failure period.

The monitoring device 100 and/or the computerized control unit 300 may monitor the person continuously, in a non-continuous manner, intermittently, or at whatever interval is desired.

Figure 15 illustrates the monitoring device 100 having an additional electrode 171 .

The additional electrode 171 is included in a housing (not shown in Figure 15 but denoted 200 in Figure 6). The additional electrode 171 is coupled to the ECG interface (not shown) within processing and transmitting module 110 via cable 174.

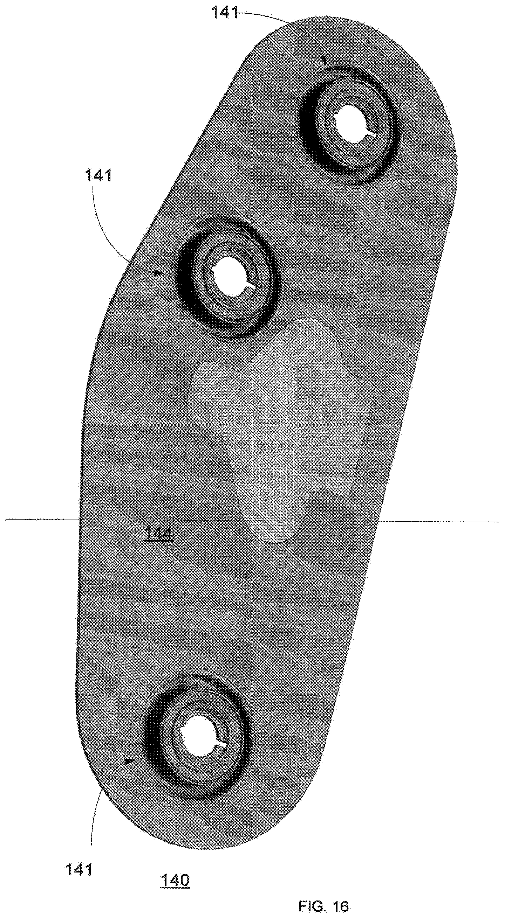

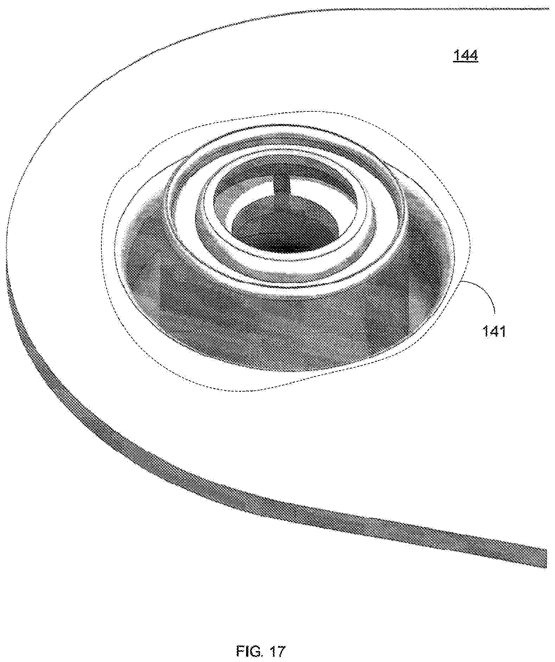

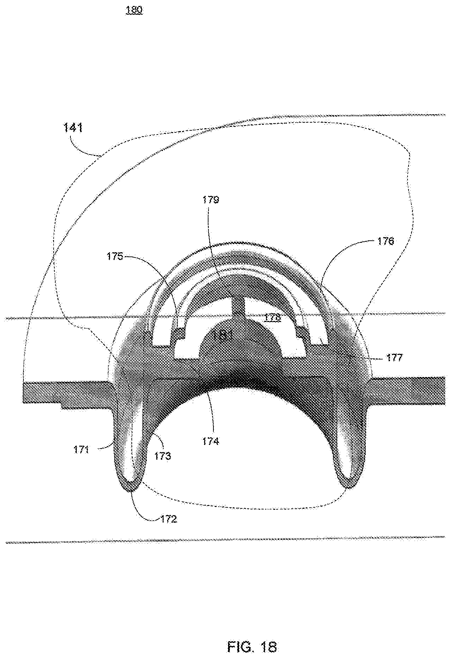

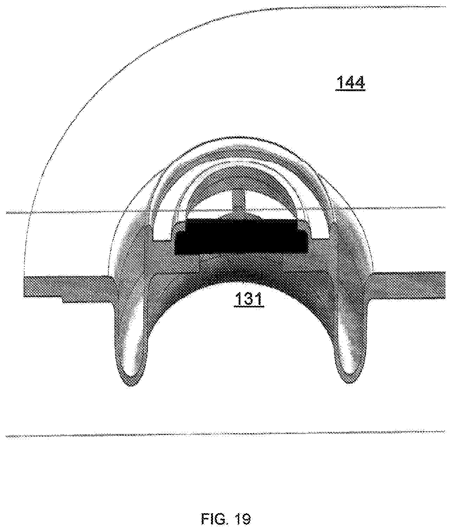

Figure 16 illustrates base layer 140 of the monitoring device 100. Figure 17 illustrates a portion of base layer 140. Figure 18 is a cross sectional view of a portion of base layer 140 of the monitoring device . Figure 19 is a cross sectional view of a portion of base layer 140 and an electrode 131 .

It has been found that rigidly connecting the electrode to the person introduces multiple noises - especially due to movements of the person, breathing of the person and the like. It has been found that suspending the electrode within an elastic housing without directly contacting the person reduces these noises dramatically.

The base layer includes a surface and one or more (for example three) electrode housings 141. Each electrode housing 141 may include (a) a trench that includes an exterior sidewall 171, a curved bottom 172, an internal sidewall 173, and a group of structural elements that includes (b) a annular segmented base 174, (c) an inner annular cover 175, (d) an external ring 176, (e) an inner trench 177 that is delimited between the inner annular cover 175 and the external ring 176. The external ring 176 and internal sidewall 173 share a same facet. The trench and the group of structural elements may be integrated with each other such that the can be made by a single mold.

Figure 18 illustrates the portion at an opposite orientation that the orientation of Figure 13. As shown, external ring and inner annular cover 175 face the person while curved bottom 172 faces the battery 121 and the top cover 110.

The electrode is positioned within a cavity 178 that is formed by the annular segmented base 174 and the inner annular cover 175. The hydrogel element 151 is placed on the inner annular cover 175 and contacts electrode 132.

The electrode housing 141 may be made from elastic material and virtually mechanically isolated from its surroundings it dumps mechanical noises that are introduced by movements of the person, person breathing and the like.

The invention may also be implemented in a computer program for running on a computer system, at least including code portions for performing steps of a method according to the invention when run on a programmable apparatus, such as a computer system or enabling a programmable apparatus to perform functions of a device or system according to the invention. The computer program may cause the storage system to allocate disk drives to disk drive groups.

A computer program is a list of instructions such as a particular application program and/or an operating system. The computer program may for instance include one or more of: a subroutine, a function, a procedure, an object method, an object implementation, an executable application, an applet, a servlet, a source code, an object code, a shared library/dynamic load library and/or other sequence of instructions designed for execution on a computer system.

The computer program may be stored internally on a non-transitory computer readable medium. All or some of the computer program may be provided on computer readable media permanently, removable or remotely coupled to an information processing system. The computer readable media may include, for example and without limitation, any number of the following: magnetic storage media including disk and tape storage media; optical storage media such as compact disk media (e.g., CD-ROM, CD-R, etc.) and digital video disk storage media; nonvolatile memory storage media including semiconductor-based memory units such as flash memory, EEPROM, EPROM, ROM; ferromagnetic digital memories; MRAM; volatile storage media including registers, buffers or caches, main memory, RAM, etc.

A computer process typically includes an executing (running) program or portion of a program, current program values and state information, and the resources used by the operating system to manage the execution of the process. An operating system (OS) is the software that manages the sharing of the resources of a computer and provides programmers with an interface used to access those resources. An operating system processes system data and user input, and responds by allocating and managing tasks and internal system resources as a service to users and programs of the system.

The computer system may for instance include at least one processing unit, associated memory and a number of input/output (I/O) devices. When executing the computer program, the computer system processes information according to the computer program and produces resultant output information via I/O devices.

In the foregoing specification, the invention has been described with reference to specific examples of embodiments of the invention. 2. The present invention is limited by the scope of the appended claims.

Those skilled in the art will recognize that the boundaries between logic blocks are merely illustrative and that alternative embodiments may merge logic blocks or circuit elements or impose an alternate decomposition of functionality upon various logic blocks or circuit elements. Thus, it is to be understood that the architectures depicted herein are merely exemplary, and that in fact many other architectures may be implemented which achieve the same functionality.

Any arrangement of components to achieve the same functionality is effectively "associated" such that the desired functionality is achieved. Hence, any two components herein combined to achieve a particular functionality may be seen as "associated with" each other such that the desired functionality is achieved, irrespective of architectures or intermedial components. Likewise, any two components so associated can also be viewed as being "operably connected," or "operably coupled," to each other to achieve the desired functionality.

Furthermore, those skilled in the art will recognize that boundaries between the above described operations merely illustrative. The multiple operations may be combined into a single operation, a single operation may be distributed in additional operations and operations may be executed at least partially overlapping in time. Moreover, alternative embodiments may include multiple instances of a particular operation, and the order of operations may be altered in various other embodiments.

Also for example, in one embodiment, the illustrated examples may be implemented as circuitry located on a single integrated circuit or within a same device. Alternatively, the examples may be implemented as any number of separate integrated circuits or separate devices interconnected with each other in a suitable manner.

Also for example, the examples, or portions thereof, may implemented as soft or code representations of physical circuitry or of logical representations convertible into physical circuitry, such as in a hardware description language of any appropriate type.

Also, the invention is not limited to physical devices or units implemented in non-programmable hardware but can also be applied in programmable devices or units able to perform the desired device functions by operating in accordance with suitable program code, such as mainframes, minicomputers, servers, workstations, personal computers, notepads, personal digital assistants, electronic games, automotive and other embedded systems, cell phones and various other wireless devices, commonly denoted in this application as 'computer systems'.

However, other modifications, variations and alternatives are also possible. The specifications and drawings are, accordingly, to be regarded in an illustrative rather than in a restrictive sense.

In the claims, any reference signs placed between parentheses shall not be construed as limiting the claim. The word 'comprising' does not exclude the presence of other elements or steps then those listed in a claim. Furthermore, the terms "a" or "an," as used herein, are defined as one or more than one. Also, the use of introductory phrases such as "at least one" and "one or more" in the claims should not be construed to imply that the introduction of another claim element by the indefinite articles "a" or "an" limits any particular claim containing such introduced claim element to inventions containing only one such element, even when the same claim includes the introductory phrases "one or more" or "at least one" and indefinite articles such as "a" or "an." The same holds true for the use of definite articles. Unless stated otherwise, terms such as "first" and "second" are used to arbitrarily distinguish between the elements such terms describe. Thus, these terms are not necessarily intended to indicate temporal or other prioritization of such elements. The mere fact that certain measures are recited in mutually different claims does not indicate that a combination of these measures cannot be used to advantage.

While certain features of the invention have been illustrated and described herein, many modifications, substitutions, changes, and equivalents will now occur to those of ordinary skill in the art. The present invention is defined solely by the scope of the appended claims.

## Claims

1. A method for monitoring electrocardiographic activity of a person, the method comprises:
performing a first set of alignment iterations for aligning a monitoring device with an electrical heart axis of the person;
wherein each alignment iteration of the first set comprises:
receiving, by a computerized control unit, electrocardiographic signals sensed by the monitoring device while the monitoring device is positioned at certain orientation while being detachably coupled to a chest of the person;
determining, by the computerized control unit and based upon the electrocardiographic signals, whether the monitoring device is aligned with the electrical heart axis of the person;
wherein the determining is responsive to amplitudes of a P wave sensed by the monitoring device and a ratio between amplitudes of a T wave sensed by the monitoring device and an R wave sensed by the monitoring device;
wherein when determining that the monitoring device is aligned with the electrical heart axis then assisting, by the computerized control unit, in a provision of an alignment indicator to the person; and
wherein when determining that the monitoring device is misaligned with the electrical heart axis then assisting, by the computerized control unit, in a provision of a request to change, by the person, an alignment of the monitoring device.

2. The method according to claim 1, comprising stopping of the performing of the first set of alignment iterations when determining that the monitoring device is aligned with the electrical heart axis.

3. The method according to claim 1, wherein the request comprises an indication about a desired change of orientation of the monitoring device.

4. The method according to claim 1, comprising determining that the monitoring device is aligned with the electrical heart axis when a peak of a P wave sensed by the monitoring device exceed an amplitude threshold and a ratio between a peak of a T wave sensed by the monitoring device and a peak of an R wave sensed by the monitoring device exceeds a ratio threshold.

5. The method according to claim 1, comprising selecting a selected orientation out of all orientations evaluated during the first set of alignment iterations in response to the determining.

6. The method according to claim 1, comprising assisting, by the computerized control unit, in a provision, to the person, of a request to (a) attach to the person an additional set of electrodes and (b) position the monitoring device below the chest of the person after each one of the alignment iterations of the first set of alignment iterations failed to align the monitoring device with the electrical heart axis.

7. A computerized control unit that comprises a transceiver, a man machine interface and a electrocardiographic processor, wherein the computerized control unit is configured to perform a first set of alignment iterations for aligning a monitoring device with an electrical heart axis of a person; wherein each alignment iteration of the first set comprises:
receiving, by the transceiver, electrocardiographic signals sensed by the monitoring device while the monitoring device is positioned at certain orientation while being detachably coupled to a chest of the person;
determining, by the electrocardiographic processor and based upon the electrocardiographic signals, whether the monitoring device is aligned with the electrical heart axis of the person;
wherein the determining, by the electrocardiographic processor, is responsive to amplitudes of a P wave sensed by the monitoring device and a ratio between amplitudes of a T wave sensed by the monitoring device and an R wave sensed by the monitoring device;
wherein when determining that the monitoring device is aligned with the electrical heart axis of the person then assisting, by at least one of the man machine interface or the transceiver, in a provision of an alignment indicator to the person; and
wherein when determining that the monitoring device is misaligned with the electrical heart axis of the person then assisting, by at least one of the man machine interface or the transceiver, in a provision of a
request to change, by the person, an alignment of the monitoring device.

8. The computerized control unit according to claim 7, wherein the man machine interface is configured to provide the alignment indicator to the person.

9. The computerized control unit according to claim 7, wherein the transceiver is configured to transmit the alignment indicator to the monitoring device.

10. The computerized control unit according to claim 7, wherein the electrocardiographic processor is configured to stop the preforming of the first set of alignment iterations when determining that the monitoring device is aligned with the electrical heart axis.

11. The computerized control unit according to claim 7, wherein the request comprises an indication about a desired change of orientation of the monitoring device.

12. The computerized control unit according to claim 7, wherein the electrocardiographic processor is configured to determine that the monitoring device is aligned with the electrical heart axis when a peak of a P wave sensed by the monitoring device exceed an amplitude threshold and a ratio between a peak of a T wave sensed by the monitoring device and a peak of a R wave sensed by the monitoring device exceeds a ratio threshold.

13. The computerized control unit according to claim 7, wherein the electrocardiographic processor is configured to select a selected orientation out of all orientations evaluated during the first set of alignment iterations in response to the determining.

14. The computerized control unit according to claim 7, wherein the man machine interface is configured to provide, to the person, of a request to (a) attach to the person an additional set of electrodes and (b) position the monitoring device below the chest of the person after each one of the alignment iterations of the first set of alignment iterations failed to align the monitoring device with the electrical heart axis.

15. The computerized control unit according to claim 7, wherein the transceiver is configured to transmit, to the monitoring device, a request to (a) attach to the person an additional set of electrodes and (b) position the monitoring device below the chest of the person after each one of the alignment iterations of the first set of alignment iterations failed to align the monitoring device with the electrical heart axis.

## Patentansprüche

1. Ein Verfahren zur Überwachung der elektrokardiographischen Aktivität einer Person, wobei das Verfahren umfasst:
Durchführen eines ersten Satzes von Ausrichtungsiterationen zum Ausrichten einer Überwachungsvorrichtung auf eine elektrische Herzachse der Person;
wobei jede Ausrichtungsiteration des ersten Satzes umfasst:
Empfangen, durch eine Computer-gestützte Steuereinheit, von elektrokardiographischen Signalen, die von der Überwachungsvorrichtung erfasst werden, während die Überwachungsvorrichtung in einer bestimmten Orientierung positioniert ist, während sie abnehmbar mit einem Brustkorb der Person gekoppelt ist;
Bestimmen, durch die Computer-gestützte Steuereinheit und basierend auf den elektrokardiographischen Signalen, ob die Überwachungsvorrichtung auf die elektrische Herzachse der Person ausgerichtet ist;
wobei die Bestimmung auf Amplituden einer von der Überwachungsvorrichtung erfassten P-Welle und auf ein Verhältnis zwischen Amplituden einer von der Überwachungsvorrichtung erfassten T-Welle und einer von der Überwachungsvorrichtung erfassten R-Welle anspricht;
wobei, wenn festgestellt wird, dass die Überwachungsvorrichtung auf die elektrische Herzachse ausgerichtet ist, dann durch die Bereitstellung eines Ausrichtungsindikators an die Person die Computer-gestützte Steuereinheit unterstützt; und
wobei, wenn festgestellt wird, dass die Überwachungsvorrichtung nicht auf die elektrische Herzachse ausgerichtet ist, dann die Computer-gestützte Steuereinheit bei einer Bereitstellung einer Aufforderung zur Änderung einer Ausrichtung der Überwachungsvorrichtung durch die Person unterstützt.

2. Verfahren nach Anspruch 1, umfassend das Anhalten der Durchführung des ersten Satzes von Ausrichtungsiterationen, wenn festgestellt wird, dass die Überwachungsvorrichtung auf die elektrische Herzachse ausgerichtet ist.

3. Verfahren nach Anspruch 1, wobei die Aufforderung einen Hinweis auf eine gewünschte Änderung der Orientierung der Überwachungsvorrichtung umfasst.

4. Verfahren nach Anspruch 1, umfassend die Bestimmung, dass die Überwachungsvorrichtung auf die elektrische Herzachse ausgerichtet ist, wenn eine Spitze einer von der Überwachungsvorrichtung erfassten P-Welle einen Amplitudenschwellenwert überschreitet und ein Verhältnis zwischen einer Spitze einer von der Überwachungsvorrichtung erfassten T-Welle und einer Spitze einer von der Überwachungsvorrichtung erfassten R-Welle einen Verhältnisschwellenwert überschreitet.

5. Verfahren nach Anspruch 1, umfassend das Auswählen einer ausgewählten Orientierung aus allen Orientierungen, die während des ersten Satzes von Ausrichtungsiterationen ausgewertet wurden, in Reaktion auf die Bestimmung.

6. Verfahren nach Anspruch 1, umfassend das Unterstützen der Computer-gestützten Steuereinheit durch Bereitstellung einer Aufforderung an die Person, (a) einen zusätzlichen Satz von Elektroden an der Person anzubringen und (b) die Überwachungsvorrichtung unter der Brust der Person zu positionieren, nachdem jede der Ausrichtungsiterationen des ersten Satzes von Ausrichtungsiterationen die Überwachungsvorrichtung nicht auf die elektrische Herzachse ausrichten konnte.

7. Computer-gestützte Steuereinheit, die einen Transceiver, eine Mensch-Maschine-Schnittstelle und einen elektrokardiographischen Prozessor umfasst, wobei die Computer-gestützte Steuereinheit so konfiguriert ist, dass sie einen ersten Satz von Ausrichtungsiterationen zum Ausrichten einer Überwachungsvorrichtung auf eine elektrische Herzachse einer Person durchführt; wobei jede Ausrichtungsiteration des ersten Satzes umfasst:
Empfangen von elektrokardiographischen Signalen durch den Transceiver, die von der Überwachungsvorrichtung erfasst werden, während die Überwachungsvorrichtung in einer bestimmten Position positioniert ist, während sie abnehmbar mit einem Brustkorb der Person gekoppelt ist,
Bestimmen, durch den elektrokardiographischen Prozessor und basierend auf den elektrokardiographischen Signalen, ob die Überwachungsvorrichtung auf die elektrische Herzachse der Person ausgerichtet ist;
wobei das Bestimmen durch den elektrokardiographischen Prozessor auf Amplituden einer P-Welle, die durch die Überwachungsvorrichtung erfasst wird, und ein Verhältnis zwischen Amplituden einer T-Welle, die durch die Überwachungsvorrichtung erfasst wird, und einer R-Welle, die durch die Überwachungsvorrichtung erfasst wird, reagiert;
wobei, wenn festgestellt wird, dass die Überwachungsvorrichtung auf die elektrische Herzachse der Person ausgerichtet ist, dann mindestens eine der Mensch-Maschine-Schnittstelle oder des Transceivers die Bereitstellung eines Ausrichtungsindikators für die Person unterstützt; und
wobei, wenn festgestellt wird, dass die Überwachungsvorrichtung nicht auf die elektrische Herzachse der Person ausgerichtet ist, dann durch mindestens eines der Mensch-Maschine-Schnittstelle oder des Transceivers die Bereitstellung einer Aufforderung an die Person, eine Ausrichtung der Überwachungsvorrichtung zu ändern, unterstützt wird.

8. Computer-gestützte Steuereinheit nach Anspruch 7, wobei die Mensch-Maschine-Schnittstelle so konfiguriert ist, dass sie der Person den Ausrichtungsindikator zur Verfügung stellt.

9. Computer-gestützte Steuereinheit nach Anspruch 7, wobei der Transceiver so konfiguriert ist, dass er den Ausrichtungsindikator an die Überwachungsvorrichtung sendet.

10. Computer-gestützte Steuereinheit nach Anspruch 7, wobei der elektrokardiographische Prozessor so konfiguriert ist, dass er die Durchführung des ersten Satzes von Ausrichtungsiterationen stoppt, wenn er feststellt, dass die Überwachungsvorrichtung auf die elektrische Herzachse ausgerichtet ist.

11. Computer-gestützte Steuereinheit nach Anspruch 7, wobei die Aufforderung eine Angabe über eine gewünschte Änderung der Orientierung der Überwachungsvorrichtung umfasst.

12. Computer-gestützte Steuereinheit nach Anspruch 7, wobei der elektrokardiographischer Prozessor konfiguriert ist, um zu bestimmen, dass die Überwachungsvorrichtung auf die elektrische Herzachse ausgerichtet ist, wenn eine Spitze einer P-Welle, die von der Überwachungsvorrichtung erfasst wird, einen Amplitudenschwellenwert überschreitet und ein Verhältnis zwischen einer Spitze einer T-Welle, die von der Überwachungsvorrichtung erfasst wird, und einer Spitze einer R-Welle, die von der Überwachungsvorrichtung erfasst wird, einen Verhältnisschwellenwert überschreitet.

13. Computer-gestützte Steuereinheit nach Anspruch 7, wobei der elektrokardiografische Prozessor so konfiguriert ist, dass er als Reaktion auf die Bestimmung eine ausgewählte Orientierung aus allen Orientierungen auswählt, die während des ersten Satzes von Ausrichtungsiterationen ausgewertet wurden.

14. Computer-gestützte Steuereinheit nach Anspruch 7, wobei die Mensch-Maschine-Schnittstelle so konfiguriert ist, dass sie der Person eine Aufforderung bereitstellt, (a) einen zusätzlichen Satz von Elektroden an der Person anzubringen und (b) die Überwachungsvorrichtung unterhalb der Brust der Person zu positionieren, nachdem jede der Ausrichtungsiterationen des ersten Satzes von Ausrichtungsiterationen die Überwachungsvorrichtung nicht auf die elektrische Herzachse ausrichten konnte.

15. Computer-gestützte Steuereinheit nach Anspruch 7, wobei der Transceiver so konfiguriert ist, dass er an die Überwachungsvorrichtung eine Aufforderung sendet, (a) einen zusätzlichen Satz Elektroden an der Person anzubringen und (b) die Überwachungsvorrichtung unter der Brust der Person zu positionieren, nachdem jede der Ausrichtungsiterationen des ersten Satzes von Ausrichtungsiterationen die Überwachungsvorrichtung nicht auf die elektrische Herzachse ausgerichtet hat.

## Revendications

1. Procédé de surveillance de l'activité électrocardiographique d'une personne, le procédé comprend :
la réalisation d'un premier ensemble d'itérations d'alignement pour aligner un dispositif de surveillance avec un axe électrique du cœur de la personne ;
dans lequel chaque itération d'alignement du premier ensemble comprend :
la réception, par une unité de commande informatisée, de signaux électrocardiographiques détectés par le dispositif de surveillance tandis que le dispositif de surveillance est positionné suivant une certaine orientation tout en étant couplé de manière détachable au thorax de la personne ;
la détermination, par l'unité de commande informatisée et sur la base des signaux électrocardiographiques, consistant à savoir si le dispositif de surveillance est aligné avec l'axe électrique du cœur de la personne ;
dans lequel la détermination est sensible à des amplitudes d'une onde P détectée par le dispositif de surveillance et à un rapport entre des amplitudes d'une onde T détectée par le dispositif de surveillance et d'une onde R détectée par le dispositif de surveillance ;
dans lequel, lorsqu'il est déterminé que le dispositif de surveillance est aligné avec l'axe électrique du cœur, l'assistance, par l'unité de commande informatisée, dans la fourniture d'un indicateur d'alignement à la personne ; et
dans lequel, lorsqu'il est déterminé que le dispositif de surveillance est désaligné par rapport à l'axe électrique du cœur, l'assistance, par l'unité de commande informatisée, dans la fourniture d'une demande pour changer, par la personne, un alignement du dispositif de surveillance.

2. Procédé selon la revendication 1, comprenant l'arrêt de la réalisation du premier ensemble d'itérations d'alignement lorsqu'il est déterminé que le dispositif de surveillance est aligné avec l'axe électrique du cœur.

3. Procédé selon la revendication 1, dans lequel la demande comprend une indication sur un changement d'orientation souhaité du dispositif de surveillance.

4. Procédé selon la revendication 1, comprenant la détermination du fait que le dispositif de surveillance est aligné avec l'axe électrique du cœur lorsqu'un pic d'une onde P détectée par le dispositif de surveillance dépasse un seuil d'amplitude et un rapport entre un pic d'une onde T détectée par le dispositif de surveillance et un pic d'une onde R détectée par le dispositif de surveillance dépasse un seuil de rapport.

5. Procédé selon la revendication 1, comprenant la sélection d'une orientation sélectionnée parmi toutes les orientations évaluées pendant le premier ensemble d'itérations d'alignement en réponse à la détermination.

6. Procédé selon la revendication 1, comprenant l'assistance, par l'unité de commande informatisée, dans la fourniture, à la personne, d'une demande pour (a) attacher à la personne un ensemble d'électrodes supplémentaire et (b) positionner le dispositif de surveillance sous le thorax de la personne après l'échec de chacune des itérations d'alignement du premier ensemble d'itérations d'alignement à aligner le dispositif de surveillance avec l'axe électrique du cœur.

7. Unité de commande informatisée qui comprend un émetteur-récepteur, une interface homme-machine et un processeur électrocardiographique, dans laquelle l'unité de commande informatisée est configurée pour réaliser un premier ensemble d'itérations d'alignement pour aligner un dispositif de surveillance avec un axe électrique du cœur d'une personne ; dans laquelle chaque itération d'alignement du premier ensemble comprend :
la réception, par l'émetteur-récepteur, de signaux électrocardiographiques détectés par le dispositif de surveillance tandis que le dispositif de surveillance est positionné suivant une certaine orientation tout en étant couplé de manière détachable au thorax de la personne ;
la détermination, par le processeur électrocardiographique et sur la base des signaux électrocardiographiques, consistant à savoir si le dispositif de surveillance est aligné avec l'axe électrique du cœur de la personne ;
dans laquelle la détermination, par le processeur électrocardiographique, est sensible à des amplitudes d'une onde P détectée par le dispositif de surveillance et à un rapport entre des amplitudes d'une onde T détectée par le dispositif de surveillance et d'une onde R détectée par le dispositif de surveillance ;
dans laquelle, lorsqu'il est déterminé que le dispositif de surveillance est aligné avec l'axe électrique du cœur de la personne, l'assistance, par au moins l'un(e) de l'interface homme-machine ou de l'émetteur-récepteur, dans la fourniture d'un indicateur d'alignement à la personne ; et
dans laquelle, lorsqu'il est déterminé que le dispositif de surveillance est désaligné par rapport à l'axe électrique du cœur de la personne, l'assistance, par au moins l'un(e) de l'interface homme-machine ou de l'émetteur-récepteur, dans la fourniture d'une demande pour changer, par la personne, un alignement du dispositif de surveillance.

8. Unité de commande informatisée selon la revendication 7, dans laquelle l'interface homme-machine est configurée pour fournir l'indicateur d'alignement à la personne.

9. Unité de commande informatisée selon la revendication 7, dans laquelle l'émetteur-récepteur est configuré pour transmettre l'indicateur d'alignement au dispositif de surveillance.

10. Unité de commande informatisée selon la revendication 7, dans laquelle le processeur électrocardiographique est configuré pour arrêter la réalisation du premier ensemble d'itérations d'alignement lorsqu'il est déterminé que le dispositif de surveillance est aligné avec l'axe électrique du cœur.

11. Unité de commande informatisée selon la revendication 7, dans laquelle la demande comprend une indication sur un changement d'orientation souhaité du dispositif de surveillance.

12. Unité de commande informatisée selon la revendication 7, dans laquelle le processeur électrocardiographique est configuré pour déterminer que le dispositif de surveillance est aligné avec l'axe électrique du cœur lorsqu'un pic d'une onde P détectée par le dispositif de surveillance dépasse un seuil d'amplitude et un rapport entre un pic d'une onde T détectée par le dispositif de surveillance et un pic d'une onde R détectée par le dispositif de surveillance dépasse un seuil de rapport.

13. Unité de commande informatisée selon la revendication 7, dans laquelle le processeur électrocardiographique est configuré pour sélectionner une orientation sélectionnée parmi toutes les orientations évaluées pendant le premier ensemble d'itérations d'alignement en réponse à la détermination.

14. Unité de commande informatisée selon la revendication 7, dans laquelle l'interface homme-machine est configurée pour fournir, à la personne, une demande pour (a) attacher à la personne un ensemble d'électrodes supplémentaire et (b) positionner le dispositif de surveillance sous le thorax de la personne après l'échec de chacune des itérations d'alignement du premier ensemble d'itérations d'alignement à aligner le dispositif de surveillance avec l'axe électrique du cœur.

15. Unité de commande informatisée selon la revendication 7, dans laquelle l'émetteur-récepteur est configuré pour transmettre, au dispositif de surveillance, une demande pour (a) attacher à la personne un ensemble d'électrodes supplémentaire et (b) positionner le dispositif de surveillance sous le thorax de la personne après l'échec de chacune des itérations d'alignement du premier ensemble d'itérations d'alignement à aligner le dispositif de surveillance avec l'axe électrique du cœur.
